# EUROPEAN PATENT SPECIFICATION

(11) **EP 2 531 046 B1**
(45) Date of publication and mention of the grant of the patent: **14.10.2015**
(21) Application number: 11701847.3
(22) Date of filing: 07.02.2011
(51) Int. Cl.: A23L 1/22, A23L 1/025, A23L 1/33, A22C 29/04, A61K 8/96, A61K 35/00, A61K 47/46, A61K 36/00

(54) **A METHOD FOR ALTERING THE FLAVOR OF A FOOD PRODUCT**
VERFAHREN ZUR VERÄNDERUNG DES GESCHMACKS EINES LEBENSMITTELS
PROCÉDÉ DE MODIFICATION DU GOÛT D'UN PRODUIT ALIMENTAIRE

(30) Priority: 05.02.2010 EP 10152841
(43) Date of publication of application: 12.12.2012
(73) Proprietor: Sense for taste, 8310 Brugge (BE)
(72) Inventor: LAHOUSSE, Bernard, B-8500 Kortrijk (BE)
(74) Representative: BiiP cvba
(86) International application number: PCT/EP2011/051722
(87) International publication number: WO 2011/095621

(56) References cited:
- EP-A1- 0 719 502
- FR-A1- 2 764 232
- US-A- 5 006 337
- US-A1- 2002 009 534
- US-A1- 2004 191 382

## Description

### FIELD OF THE INVENTION

The present invention relates to a method for altering the flavor of a food product comprising impregnating a substance into a food product by exposing the food product to a pressure from 1000 to 6000 bar together with a compound containing the substance.

### BACKGROUND OF THE INVENTION

Impregnating or marinating of food in wine, spices, etc. at room temperature or below has been known for centuries. One huge disadvantage of this classical method of impregnation is the fact that this process is very slow, and thus very time-consuming. For example, in order to sufficiently impregnate meat with a specific flavor, it must be marinated for 2 to 3 days. Otherwise, the flavor isn't sufficiently absorbed into the meat.

Other examples of classical impregnation of food products are the pickling of meat or fish or the candying of fruits. Similar to marinating, these methods are also very slow and time-consuming.

More recently, impregnation of flavoring substances in food products such as meat, fish, has been carried out by vacuum techniques, wherein a food product is placed in a vacuum for a certain period of time, together with a liquid. For example, an apple placed in an amount of red beetroot juice under vacuum pressure, will result in a red apple that is completely impregnated with the flavor of the red beetroot. Several examples of this technique are described by Frito et al, 1994 (Vacuum osmotic dehydratation, Process optimization and minimal processing of foods).

However, a major disadvantage of this technique is that it can not be applied on all food products, and its efficiency is variable and very much depending of the specific application in which it is used. For example, shellfish cannot be impregnated with this technique because the shellfish meat cannot withstand a vacuum and will be destroyed.

Another impregnating technique is injecting substance with needles. For example, brine is often injected in meat as described in US 5934187, or if applied on a larger, more industrial scale in WO2008135610.

A disadvantage of this technique however is the non-equal spreading of the fluid containing a certain aroma or flavor.

Another disadvantage is the fact that this technique can not be applied to food products suffering from increased fluid loss after injecting, such as for example shellfish.

Still another disadvantage is that, to allow injecting a fluid containing a substance, such fluid has to be prepared first, resulting in additional process steps, increased process time and increased production cost.

Other examples of impregnating food products are described in US2002/0009534 and in EP0719502. In US2002/0009534 shellfish is immersed in a pressurized fluid wherein substances like salt, lemon juice, hot sauces are added. In EP0719502, fruit juice is flavored by adding to the pressurized fluid a substance, either synthesized from natural products, either synthetic.

A disadvantage of the above methods is again that the substances have to be prepared first (by refinement, squeezing, chemical synthesis, etc.) and added to the pressurized fluid, resulting in additional process steps, increased process time and increased production cost.

Considering the above drawbacks, it is an object of the present invention to provide a method for altering the flavor of a food product, which is less suffering from variable efficiency, less suffering from application dependent performance, less suffering from unequal spreading of the aroma or flavor, and applicable to all food products.

It is an object of the present invention to provide a method for altering the flavor of a food product wherein the user is not forced to first prepare substances to be impregnated in the food product.

Another object of the present invention is to provide a method for altering the flavor of a food product wherein the user is not forced to use synthesized substances, or synthetic aromatizing substances.

It is another object of the present invention to provide a method having the ability to not merely flavoring the food product, but rather clearly alter the flavor of the food product.

Another object of the present invention is to provide a method allowing selecting the flavor to which the original flavor should alter.

### SUMMARY OF THE INVENTION

The present invention is directed to a method for altering the flavor of a food product comprising impregnating a substance into a food product by exposing the food product to a pressure from 1000 to 6000 bar together with a solid compound containing the substance and extracting the substance from the solid compound simultaneously with the step of impregnating, characterized in that the method further comprises selecting the substance to be extracted from the solid compound, determining the pressure suitable for extracting the selected substance, and at least partially immersing the solid compound in a liquid selected for extracting the selected substance.

In addition, the present invention is directed to the use of such method for the preparation of food supplements, vitamin supplements, therapeutic compositions, or cosmetic compositions.

### DESCRIPTION OF THE INVENTION

In the context of the present invention, altering the flavor of a food product is understood as comprising not only merely flavoring the food product, but optionally even changing the original flavor of the food product into the flavor of the solid compound or of the substance extracted thereof.

In the context of the present invention, a substance is understood as a substance chosen from the group of aromas, aromatic molecules, colorings agents, softening agents, enzymes, gelating agents food additives, taste molecules, health improving components, texture changing components, vitamins, minerals, antimicrobial substances, and combinations of two or more thereof.

In the context of the present invention, a solid compound is understood as a solid food product, such as vegetables, fruit, meat, fish, shell fish, crustacean, nuts, cheese, cereals, spices, herbs, or as a non-food product such as wood, plants, or flowers.

According to a first embodiment of the present invention a method for altering the flavor of a food product is provided comprising impregnating a substance into a food product by exposing the food product to a pressure from 1000 to 6000 bar together with a solid compound containing the substance and extracting the substance from the solid compound simultaneously with the step of impregnating, characterized in the method further comprises selecting the substance to be extracted from the solid compound, determining the pressure suitable for extracting the selected substance, and at least partially immersing the solid compound in a liquid selected for extracting the selected substance.

The inventors have surprisingly found that it is possible to alter the flavor of a food product in a very short time span by simultaneously extracting a substance from a solid compound and impregnating that substance into a food product at a pressure from 1000 to 6000 bar.

A first advantage of such method is that it may be less suffering from variable efficiency, less suffering from application dependent performance, less suffering from unequal spreading of the aroma or flavor, and applicable to all food products. Another advantage of such method may be that the user is not forced to first prepare substances to be impregnated in the food product. Moreover, he is not forced to use synthesized substances, or synthetic aromatizing substances. On the contrary, he may directly use raw, natural, even completely untreated solid compounds containing the substance to be impregnated. For example, to impregnate ethyl dodecanoate in a food product, he may use natural raw coconut, or to impregnate beta-damascenone in a food product, he may use a rose.

A further advantage of such method may be that it has the ability to not merely flavoring the food product, but optionally clearly alter the flavor of the food product.

In embodiment in accordance with the present invention, the food product may be a solid or a liquid.

Such liquid food product may for example be water, bouillon, fumet, vegetable juices, fruit juices, alcoholic drinks, seawater, sugar syrup, oil, molten chocolate, water extracts from cheese, condiments, fond, alcoholic drinks, spirits, liquors, beer, wine, soft drinks, or energy drinks.

Such solid food product may be shell-fish, fish, meat, vegetables, fruit, nuts, cereals, eggs, fungi, mushrooms, coffee, dairy, plants, herbs, spices, seaweeds, seeds, flowers and derivates thereof and combinations thereof.

A preferred application of a method in accordance with the present invention may be altering the flavor of shell-fish, in particular bivalves. Since the meat of bivalves such as oysters and mussels is completely shut off from the outside world, it is very difficult to impregnate them with substances. Often, the only way to transfer some taste to a bivalve is to open them, marinate them or to process them at elevated temperature with a sauce. By using a method according to the present invention, shell fish such as bivalves are impregnated with substances without opening them before exposure to high pressure.

The food products may also be food supplements or vitamin supplements, or food products used in therapeutic or cosmetic compositions.

The high pressure used in a method in accordance with the present invention may be between 1000 - 6000 bar, preferably between 2000 - 6000, more preferably between 3000 - 6000 bar, most preferably between 4000- 6000 bar.

The food product together with the solid compound may be exposed to a high pressure for a period of at least one second, preferably at least one minute, more preferably at least 3 minutes, but preferably no more than 5 minutes.

For very sensible food products, a time period of 1 second may be applied in order not to damage the food product. Generally speaking, 1 minute will be sufficient for a food product to absorb a substantial part of the substances from the solid compound. Above 5 minutes, the amount of substances absorbed by the food product may reduce drastically such that no additional benefits are gained from applying longer exposure times.

As a consequence, a product with a low shelf life, such as fresh shell-fish, can also be impregnated given the fact that the time to impregnate may be reduced from a couple of days to a couple of minutes, or even seconds.

It is found by the inventors that the pressure used may have an influence on either which and which amount of substance is extracted from the solid compound, either which and which amount of substance is impregnated into the food product. For example, upon increasing the pressure when using star anise as solid compound, the food product is flavored with increasing black tea flavor.

It appears that seeking for an optimum between maximum flavor transfer from solid compound to food product, and the amount of tannins, or bitter or sour substances transferred results in an optimum pressure. For example, in case the solid compound is mushroom, ginger, pineapple, the optimum pressure is about 4000 bar, whereas for coriander, star anise, orange, natural vanilla, the optimum pressure is about 5000 bar, and for celery about 3000 bar.

The food product may be packed together with the solid compound in a packaging, preferably a vacuum packaging before exposure to high pressure, enhancing substance transfer between solid compound and food product.

Preferably, in case the food product is a liquid, the solid compound is at least partially immersed in the liquid. In case the food product is a solid, preferably both solid compound and food product are at least partially immersed in a liquid.

Preferably, the solid compound and the solid food product are in close proximity, by as understood by a person skilled in the art, a method in accordance with the present invention may be thought of wherein the solid compound and the solid food product are pressurized together, each in its own process cavity but both in contact with the liquid in which they are at least partially immersed.

The liquid selected for extracting the selected substance and in which the solid compound may be partially immersed may be either the food product itself, either a liquid used, without being bound by any theory, as a solvent for the selected substance. The choice of liquid may have an influence on either which and which amount of substance is extracted from the solid compound, either which and which amount of substance is impregnated into the food product. For example, upon using star anise as solid compound, the food product is flavored with respectively more anise flavor, or more black tea flavor by using alcohol in stead of water. It appears that seeking for an optimum between maximum flavor transfer from solid compound to food product, and the amount of tannins transferred results in an optimum pressure.

Liquids selected for extracting the selected substance may be water, bouillon, alcohol, or any liquid mentioned earlier.

A method in accordance with the present invention allows the building-up of a taste profile of a desired final product. By using the correct solid compound and the appropriate pressures and liquids to immerse the solid compound in, and by making suitable combinations or sequences thereof, the desired taste profile may be reached.

A method in accordance with the present invention may be used in a sequence of impregnating steps. For example, an orange as solid compound may be pressurized in water at 5000 bar, and subsequently after removing the orange, cockles may be impregnated with the obtained orange flavored water at 6000 bar.

A method in accordance with the present invention may additionally be used also for reducing or eliminating pathogenic organisms in the food product, particular Vibrio pathogens in oysters.

Such method may also additionally used for adjusting or improving the texture with respect to bite and volume of the food product.

Such method may also additionally be used to impregnate anti-microbial and/or therapeutic substances into liquid food products, or even directly into solid food products. For example, using a combination of star anise and elderberry as solid compound may result in impregnation of shikimic acid and flavonoides, which are substances known to have therapeutic power, for example in case of influenza.

Such method may also additionally used for adjusting or improving characteristics such as shelf live, color stability, or oxidation speed of food products by using solid compounds containing anti-oxidation agents, anti-coloring agents, etc.

Different experiments have been conducted on a high pressure device from NC Hyperbaric with a 55 liter content per batch, at a temperature of 5-30°C, with a maximum pressure of 600 MPa (= 6000 bar). Examples are given below.

### Example 1:

It is possible to transfer the taste of ginger to an oyster by simple placing a piece of ginger near the shell of a fresh closed oyster in water at 5000 bar for 1 minute. Result: The taste of the ginger will completely be absorbed in the oyster without the oyster losing its natural flavor.

### Example 2:

A fresh oyster was packed vacuum together with a piece of kiwi in water. The oyster was kept at 6000 bar for 1 minute.

Result: the oyster has a soft taste of kiwi combined with the natural taste of a fresh oyster.The texture has slightly evolved because of the high pressure so that an oyster was obtained with a little more bite,

### Example 3:

A piece of lamb meat of 100 g was packed together with 5 grams of seaweed and 20 grams of bouillon. The meat was kept at 6000 bar for one minute.

Result: the meat is fully impregnated with the taste of the seaweed.

### Example 4:

Raw cucumber and cockles are immersed in water for 1 minute at 5000 bar.

Result: the cockles have a strong cucumber taste which is surprising because naturally, raw cucumber tastes very weak.

### Example 5

Water was packed together with seaweed and a piece of lamb meat. This mixture was treated with a pressure of 6000 bar for five minutes.

Result: the water tasted like lamb and seaweed. This liquid could be used as a basis for sauces.

### Example 6:

Wine was packed together with lemongrass. The mixture was treated with a pressure of 6000 bar for five minutes.

Result: the wine had the aromas of lemongrass.

### Example 7:

Fresh grapes were packed together with white wine, apricot, cinnamon, roses. The mixture was treated at 6000 bar for 30 seconds.

Result: the grapes stayed crispy, gained in volume and the taste was clearly still grape, but enriched with rose, cinnamon, apricot flavors and wine.

### Example 8:

Wine was packed with lemongrass, thyme, apricot and pressurized for 5 minutes at 6000 bar. The wine was enriched with all the flavors, but was also thickened by components from the apricot, more like a dessert wine.

### Example 9:

Celery stalks were added to a bag filled with water and kiwi fruit. By applying 6000 bar during 4 minutes, the stalks gained in crispness and tasted like celery with small hints of kiwi.

### Example 10:

Cucumber was packed with vodka and treated for 4 minutes at 6000 bar. The vodka obtained a very rich cucumber taste and flavor.

### Example 11:

Strawberries were combined with milk and pressurized 4 minutes at 4000 bar. The colour from the strawberry together with the flavor were extracted into the milk. The colour and the flavor were very intense. The strawberry lost partially its colour and most of its flavor.

### Example 12:

Fresh bananas were combined with molten chocolate. They were processed warm at 6000 bar during 5 minutes. The chocolate contains very distinct banana flavors after the process and after the bananas were removed from the chocolate.

### Example 13:

Fresh cherries were packed with star anise, orange peel and vodka, and pressurized for 4 minutes at 5000 bar.

Result: cherries infused with alcohol, orange flavor and anise flavor.

### Example 14:

Cherry tomatoes were combined with cucumber, orange peel, waste material of shrimps and salted water and pressurized for 4 minutes at 6000 bar. The cherry tomatoes contained the flavors, especially the peel of the tomato.

### Example 15:

In whiskey, the following aroma's can be distinguished.
- 3-methylbutanal: malt
- Vanillin: vanilla
- Guaiacol: coffee
- Maltol: larix wood
- Eugenol: cloves
- Oak lactone: licorice
- Acetaldehyde: orange peel
- Furaneol: Tamarinde
- Furfural: pineapple
- Ethyl octanoate: plum
- Ethyl hexanoate: apple
- 4-vinylguaiacol: lovage root
- 2,3-butanedione: strawberry
- Ethyl dodecanoate: coconut
- Ethyl decanoate: plum
- 2-phenylethanol: grapes
- Isoamyl acetate: banana
- Beta-damascenone: rose

By using the corresponding natural solid compound mentioned next to each aroma, and using the appropriate pressures and liquids to immerse the solid compound in, and by making suitable combinations or suitable sequences thereof, a taste profile of whiskey may be obtained, which may be used in improving the quality of lower quality whiskeys.

Likewise applications may be in using wood, vanillin, etc. for improving the taste of wines, improving the taste of balsamic vinegar, improving the taste of gin, etc.

## Claims

1. A method for altering the flavor of a food product comprising impregnating a substance into a food product by exposing the food product to a pressure from 1000 to 6000 bar together with a solid compound containing the substance and extracting the substance from the solid compound simultaneously with the step of impregnating, **characterized in that** the method further comprises selecting the substance to be extracted from the solid compound, determining the pressure suitable for extracting the selected substance, and at least partially immersing the solid compound in a liquid selected for extracting the selected substance.

2. A method according to claims 1, **characterized in that** the food product is a solid or a liquid.

3. A method according to claims 1 or 2, wherein the liquid selected for extracting the selected substance is the food product itself.

4. A method according to claims 1 to 3 **characterized in that** the food product is chosen from a group comprising shell-fish, fish, meat, vegetables, fruit, nuts, cereals, eggs, fungi, mushrooms, coffee, dairy, plants, herbs, spices, seaweeds, seeds, flowers and derivates thereof and combinations thereof, or from a group comprising water, bouillon, fumet, vegetable juices, fruit juices, alcoholic drinks, seawater, sugar syrup, oil, molten chocolate, water extracts from cheese, condiments, fond, alcoholic drinks, spirits, liquors, beer, wine, soft drinks, or energy drinks.

5. A method according to claim 4 **characterized in that** the food product is chosen from the group of mollusks or bivalves.

6. A method according to any one of claims 1 to 5, **characterized in that** the solid compound is chosen from the group comprising food products, vegetables, fruit, meat, fish, shell fish, crustacean, nuts, cheese, cereals, spices, herbs, plants, wood, flowers, or a combination thereof.

7. A method according to any one of claims 1 to 6, **characterized in that** the substance is chosen from the group of aromas, aromatic molecules, colorings agents, food additives, taste molecules, health improving components, texture changing components, vitamins, minerals and combinations of two or more thereof.

8. A method according to any one of claims 1 to 7, **characterized in that** the high pressure is between 4000 - 6000 bar.

9. A method according to any one of claims 1 to 8, **characterized in that** the food product, together with the solid compound is exposed to a high pressure for a period from 1 second up to 5 minutes.

10. A method according to any one of claims 1 to 9, **characterized in that** the food product is packed together with the solid compound in a packaging.

11. A method according to claim 10, **characterized in that** the food product is packed together with the solid compound in a vacuum packaging.

12. Use of the method according to any of the above claims for the preparation of food supplements, vitamin supplements, therapeutic compositions, or cosmetic compositions.

## Patentansprüche

1. Eine Methode zur Veränderung des Geschmacks eines Lebensmittelprodukts umfassend die Imprägnierung einer Substanz in ein Lebensmittelprodukt durch Aussetzen des Lebensmittelprodukts an einen Druck von 1000 bis 6000 bar zusammen mit einer soliden Verbindung, die die Substanz enthält, und Extrahieren der Substanz aus der soliden Verbindung gleichzeitig mit dem Imprägnierungsschritt, **dadurch gekennzeichnet, dass** die Methode des Weiteren umfasst die Auswahl der Substanz, die aus der soliden Verbindung extrahiert werden soll, Bestimmung des geeigneten Drucks zum Extrahieren der gewählten Substanz, und zumindest ein teilweises Eintauchen der soliden Verbindung in eine gewählte Flüssigkeit zum Extrahieren der gewählten Substanz.

2. Eine Methode nach Anspruch 1, **dadurch gekennzeichnet, dass** das Lebensmittelprodukt ein Feststoff oder eine Flüssigkeit ist.

3. Eine Methode nach den Ansprüchen 1 oder 2, wobei die gewählte Flüssigkeit zum Extrahieren der gewählten Substanz das Lebensmittelprodukt selbst ist.

4. Eine Methode nach den Ansprüchen 1 bis 3 **dadurch gekennzeichnet, dass** das Lebensmittelprodukt gewählt wird aus einer Gruppe umfassend Schalentiere, Fisch, Fleisch, Gemüse, Obst, Nüsse, Zerealien, Eier, Fungus, Pilze, Kaffee, Molkereiprodukte, Pflanzen, Kräuter, Gewürze, Meeresalgen, Samen, Blüten und deren Derivaten und Kombinationen hieraus, oder aus einer Gruppe umfassend Wasser, Fleischbrühe, Wildextrakt, Gemüsesäfte, Obstsäfte, Alkoholgetränke, Meerwasser, Zuckersirup, Öl, gemahlene Schokolade, Wasserextrakte aus Käse, Gewürze, Fond, Alkoholgetränke, Spirituosen, Liköre, Bier, Wein, alkoholfreie Getränke oder Energiegetränke.

5. Eine Methode nach Anspruch 4, **dadurch gekennzeichnet, dass** das Lebensmittelprodukt aus der Gruppe der Molluske oder Muscheln gewählt wird.

6. Eine Methode nach einem der Ansprüche 1 bis 5, **dadurch gekennzeichnet, dass** die solide Verbindung aus der Gruppe umfassend Lebensmittelprodukte, Gemüse, Obst, Fleisch, Fisch, Schalentiere, Krustentiere, Nüsse, Käse, Zerealien, Gewürze, Kräuter, Pflanzen, Holz, Blüten, oder einer Kombination hieraus gewählt wird.

7. Eine Methode nach einem der Ansprüche 1 bis 6,**dadurch gekennzeichnet, dass** die Substanz gewählt wird aus der Gruppe der Aromas, aromatischen Moleküle, Farbstoffe, Lebensmittelzusätze, Geschmacksmoleküle, gesundheitsverbessernden Komponenten, texturverändernden Komponenten, Vitamine, Minerale und Kombinationen von zwei oder mehr hieraus gewählt wird.

8. Eine Methode nach einem der Ansprüche 1 bis 7, **dadurch gekennzeichnet, dass** der Hochdruck zwischen 4000 und 6000 bar beträgt.

9. Eine Methode nach einem der Ansprüche 1 bis 8, **dadurch gekennzeichnet, dass** das Lebensmittelprodukt zusammen mit der soliden Verbindung für einen Zeitraum von 1 Sekunde bis 5 Minuten einem Hochdruck ausgesetzt wird.

10. Eine Methode nach einem der Ansprüche 1 bis 9, **dadurch gekennzeichnet, dass** das Lebensmittelprodukt zusammen mit der soliden Verbindung in einer Verpackung verpackt wird.

11. Eine Methode nach Anspruch 10, **dadurch gekennzeichnet, dass** Lebensmittelprodukt zusammen mit der soliden Verbindung in einer Vakuumverpackung verpackt wird.

12. Verwendung der Methode nach einem der obigen Ansprüche für die Zubereitung von Lebensmittelergänzungen, Vitaminergänzungen, therapeutischen Zusammensetzungen oder kosmetischen Zusammensetzungen.

## Revendications

1. Procédé de modification du goût d'un produit alimentaire comprenant l'imprégnation par une substance d'un produit alimentaire en exposant le produit alimentaire à une pression de 1000 à 6000 bars conjointement avec un composé solide contenant la substance et l'extraction de la substance du composé solide simultanément avec l'étape d'imprégnation, **caractérisé en ce que** le procédé comprend en outre les étapes consistant à choisir la substance à extraire du composé solide, déterminer la pression convenant à l'extraction de la substance choisie et immerger au moins en partie le composé solide dans un liquide choisi pour extraire la substance choisie.

2. Procédé selon la revendication 1, **caractérisé en ce que** le produit alimentaire est un solide ou un liquide.

3. Procédé selon la revendication 1 ou la revendication 2, dans lequel le liquide choisi pour extraire la substance choisie est le produit alimentaire lui-même.

4. Procédé selon les revendications 1 à 3, **caractérisé en ce que** le produit alimentaire est choisi dans un groupe comprenant des coquillages, du poisson, de la viande, des légumes, des fruits, des noix, des céréales, des oeufs, des moisissures, des champignons, du café, des produits laitiers, des végétaux, des herbes, des épices, des algues, des semences, des fleurs et leurs dérivés ainsi que leurs combinaisons, ou dans un groupe comprenant l'eau, un bouillon, un fumet, des jus de légumes, des jus de fruits, des boissons alcoolisées, de l'eau de mer, du sirop de sucre, de l'huile, du chocolat fondu, des extraits aqueux de fromage, des condiments, des fonds, des boissons alcoolisées, des spiritueux, des liqueurs, de la bière, du vin, des sodas ou des boissons énergisantes.

5. Procédé selon la revendication 4, **caractérisé en ce que** le produit alimentaire est choisi dans le groupe des mollusques ou des bivalves.

6. Procédé selon l'une quelconque des revendications 1 à 5, **caractérisé en ce que** le composé solide est choisi dans le groupe comprenant des produits alimentaires, des légumes, des fruits, de la viande, du poisson, des coquillages, des crustacés, des noix, du fromage, des céréales, des épices, des herbes, des plantes, du bois, des fleurs ou une de leurs combinaisons.

7. Procédé selon l'une quelconque des revendications 1 à 6, **caractérisé en ce que** la substance est choisie dans le groupe des aromes, des molécules aromatiques, des agents colorants, des additifs alimentaires, des molécules de goût, des composants qui renforcent la santé, des composants de changement de texture, des vitamines, des minéraux et des combinaisons de deux ou plusieurs d'entre eux.

8. Procédé selon l'une quelconque des revendications 1 à 7, **caractérisé en ce que** la pression élevée se situe entre 4000 et 6000 bars.

9. Procédé selon l'une quelconque des revendications 1 à 8, **caractérisé en ce que** le produit alimentaire est exposé conjointement avec le composé solide à une pression élevée pendant une période de 1 seconde à 5 minutes.

10. Procédé selon l'une quelconque des revendications 1 à 9, **caractérisé en ce que** le produit alimentaire est conditionné conjointement avec le composé solide dans un emballage.

11. Procédé selon la revendication 10, **caractérisé en ce que** le produit alimentaire est conditionné conjointement avec le composé solide dans un emballage sous vide.

12. Utilisation du procédé selon l'une quelconque des revendications précédentes pour la préparation de suppléments alimentaires, de suppléments vitaminés, de compositions thérapeutiques ou de compositions cosmétiques.
